# EUROPEAN PATENT APPLICATION

(11) **EP 4 378 511 A1**
(43) Date of publication of application: **05.06.2024**
(21) Application number: 22210886.2
(22) Date of filing: 01.12.2022
(51) Int. Cl.: A61M 25/00, A61M 5/178, A61L 101/32

(54) **A CATHETER WITH AN ANTIMICROBIAL EXTERIOR COATING**

(71) Applicant: Labo Groep B.V., 5051 ZL Goirle (NL)
(72) Inventor: Zwart, Gerrit, 5051 ZL Goirle (NL)
(74) Representative: IPecunia

(57) **Abstract**

A method and a system for preparing an externally coated catheter (10). A sterile outer packaging (1) contains therein a catheter (10) received in a dispenser tube (15). A manually operable injector (30) is filled with a liquid antimicrobial coating substance. The dispenser tube (15) is provided with a connector port member (20), and the injector (30) is provided with a mating connector member (41). The liquid antimicrobial coating substance is dispended from the injector (30) so as to introduce the liquid antimicrobial coating substance into the dispenser tube (15) thereby coating the exterior of the catheter (10) with the liquid antimicrobial coating substance prior to removal of the coated catheter (10) from the dispenser tube (15).

## Description

The present invention relates to the field of catheters that are to be introduced into a patient.

In the field it is known to provide a catheter on the exterior thereof with an antimicrobial coating, e.g. in view of reduction of infections.

In the prior art, the antimicrobial coating is applied to the exterior of the catheter during production of the catheter. The antimicrobial coated catheter is then received in a dispenser tube to protect the fragile catheter. The assembly is then packaged in an outer packaging, e.g. is subject to a sterilizing treatment whilst packaged in the outer packaging. Examples of catheters, dispenser tubes, and outer packaging are, for example, disclosed in EP782868, US2004/0055919, and EP1060757.

It is noted that is also known to coat the interior of the catheter, e.g. with an antimicrobial coating. Whilst the present invention is related to an approach for coating the exterior, the catheter to be coated exteriorly may have already been provided with an internal coating during production and/or may be made of a material that includes an antimicrobial agent.

The use of propolis in the coating of medical implants, in particular for stents, is discussed in US2007/0003591. This document provides an extensive discussion of propolis, including its composition, variants thereof, and possible effects when applied as a coating for an implant.

The present invention aims to provide a new approach to provide a catheter having its exterior coated with an antimicrobial coating, in particular a coating having propolis as an agent.

To this end, the invention proposes a catheter system according to claim 1.

The catheter system comprises an outer packaging containing therein a catheter received in a dispenser tube, the catheter having an exterior. The catheter system further comprises a manually operable injector which is filled with a liquid antimicrobial coating substance.

In the inventive system, the dispenser tube is provided with a connector port member and the injector is provided with a mating connector member, which is connectable or connected to the connector port member.

The inventive system is configured to allow for the dispensing of the liquid antimicrobial coating substance from the injector so as to introduce the liquid antimicrobial coating substance into the dispenser tube thereby coating the exterior of the catheter with the liquid antimicrobial coating substance prior to removal of the coated catheter from the dispenser tube.

In the inventive approach, the liquid antimicrobial coating substance can be applied in a simple and reliable manner to the exterior of the catheter, and preferably is applied only shortly before the actual introduction of the externally coated catheter into the patient. This allows to keep the liquid antimicrobial coating substance confined in the injector until its application on the exterior of the catheter, which may be beneficial in view of shelf-life and/or properties over time of the coating substance compared to the prior art approach wherein the external coating has already been applied during production of the catheter. Also, due to its late stage application, the external coating can be applied in a fairly liquid state and/or in an abundance on the exterior of the catheter which is difficult/impossible when providing the external coating during production as the coating will or may then wear off during storage and/or transportation of the packaged catheter. The inventive approach may in practice allow for the formation of a cuff of antimicrobial coating substance at the site of entry into the patient, as some of the coating substance will be stripped from the catheter at this location during its introduction into the patient. The cuff may form a barrier about the catheter at the entry site for pathogens, bacteria, etc.

In a preferred embodiment, the liquid antimicrobial coating substance comprises or is primarily composed of propolis. The propolis containing external coating of the catheter may provide enhanced protection against catheter related infections.

In an embodiment, the connector port member is provided at an end of the dispenser tube. For example, the connector port is provided at a rear end of the dispenser tube with the catheter being removed via the front end of the dispenser tube. In another embodiment, the connector port is located somewhere along the length of the dispenser tube, e.g. in a central region of the dispenser tube. For example, the connector port member is combined with, e.g. integrated with, a coiling clip that is provided to keep the dispenser tube in a coiled configuration, at least whilst contained in the outer packaging.

Preferably, the injector is initially separated from the dispenser tube receiving the catheter therein, so that in use of the system the injector is then to be connected to the dispenser tube. In another embodiment, the injector is already connected to the dispenser tube and in this configuration packaged in the outer packaging.

In an embodiment, the connector port member comprises a Luer fitting, e.g. a female Luer fitting, e.g. a female Luer - lock fitting. For example, the port member is of plastic and has been heat sealed to the dispenser tube. In an embodiment, the injector comprises a mating Luer fitting, e.g. a male is a Luer fitting, e.g. a male Luer - lock fitting.

For example, the connector port member has a closure, e.g. a removable closure, e.g. a cap. In another embodiment, the connector port member has a pierceable or breakable closure, that is pierced or broken upon making the connection. In other embodiments, a closure associated with the port member is to be removed or opened first, ahead of a connecting step. The closure may be directly engaged with the port member. In other embodiments, the connector port member lacks any form of closure.

In an embodiment, the injector comprises a first chamber that is filled with the liquid antimicrobial coating substance and a second chamber that is filled that is filled with a solvent and/or diluent, e.g. an alcohol.

In an embodiment, the injector is a syringe, e.g. a double barrel syringe having a first chamber filled with the liquid antimicrobial coating substance and a second chamber filled with a solvent and/or diluent.

In an embodiment, the injector is configured to dispense from both the first and second chamber simultaneously so as to enhance the mixing of the coating substance with the solvent/diluent.

In an embodiment, the injector comprises a static mixer connected or connectable to the first and second chambers at an inlet of the static mixer, wherein an outlet of the static mixer is provided with the mating connector member that is connectable or connected to the port member of the dispenser tube.

In an embodiment, the injector holds between 10 ml and 30 ml of the liquid antimicrobial coating substance.

In an embodiment, the outer packaging comprises a flexible pouch in which the catheter received in the dispenser tube is packaged. Other embodiments of the outer packaging are also envisaged, e.g. with a tray covered by a foil.

In an embodiment, the manually operable injector filled with a liquid antimicrobial coating substance is initially also contained within the outer packaging. This allows for sterilization of the injector and the substance therein together with all other components of the system within the outer packaging, at least including the catheter which is received in the dispenser tube. For example, sterilization involves irradiating the packaged system.

The present invention also relates to a method for preparing an externally coated catheter, wherein a system as described herein is provided, and wherein the method comprises:
- opening the outer packaging, e.g. the sterilized outer packaging,
- possibly, connecting the injector with the mating connector member thereof to the connector port member of the dispenser tube,
- operating the injector so as to dispense the liquid antimicrobial coating substance from the injector so as to introduce the liquid antimicrobial coating substance into the dispenser tube thereby coating the exterior of the catheter with the liquid antimicrobial coating substance prior to removal of the coated catheter from the dispenser tube,
- removing the coated catheter from the dispenser tube.

The invention will now be discussed with reference to the drawings. In the drawings:

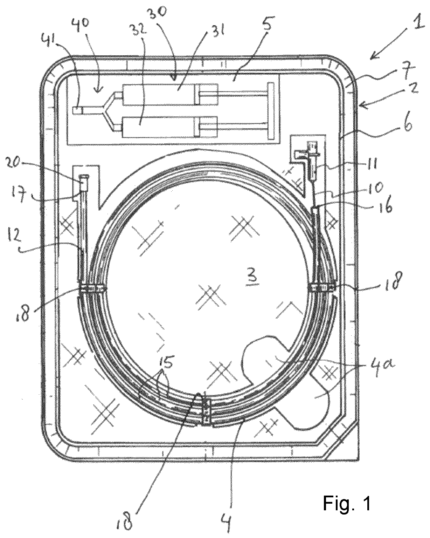

- fig. 1 shows schematically an example of a catheter system according to the invention.

Figure 1 shows an example of a catheter system according to the invention.

The system comprises an outer packaging 1, here embodied with a tray 2 having a bottom 3 which is provided with recessed storage areas 4, 5 and having a peripheral wall 6 with a peripheral upper flange 7. A foil (not shown) is hermetically sealed on the tray 2 at the flange 7.

The system further comprises a catheter 10 which is received in a dispenser tube 15.

The catheter 10 has a manipulator member 11 at a proximal end thereof. The catheter has an active distal end 12.

The dispenser tube 15 has a front end 16 and a rear end 17. The catheter 10 is to be removed from the dispenser tube 15 via the front end 16 thereof.

The dispenser tube 15 is coiled, here in a spiral, as is known in the art. Coiling clips 18 maintain the tube 15 and the catheter 10 received therein in the coiled configuration.

The coiled dispenser tube 15 and catheter 10 are located in recessed storage area 4 of the tray 2 with grip sections 4a.

The dispenser tube 15 is provided with a connector port member 20, here at the rear end 17 thereof. Preferably, the port member 20 comprises a Luer fitting, e.g. a female Luer fitting, e.g. a female Luer - lock fitting.

The system also comprises a manually operable injector 30 which is filled with a liquid antimicrobial coating substance. As shown by way of example, the injector 30 is located in recessed storage area 5 of the tray 2.

In the depicted example, the injector 30 comprises a double barrel syringe having a first chamber 31 which is filled with the liquid antimicrobial coating substance and a second chamber 32 which is filled with a solvent and/or diluent for the substance, e.g. alcohol.

The injector 30 is configured to dispense from both the first and second chambers 31, 32 simultaneously.

In another embodiment, the injector 30 is embodied as a single barrel syringe containing a liquid antimicrobial coating substance. Other injectors 30 are also envisaged, e.g. as a squeezable capsule containing a liquid antimicrobial coating substance.

In the depicted example, injector 30 further comprises a static mixer 40 which is connected or connectable to the first and second chambers 31, 32 at an inlet of the static mixer. An outlet of the static mixer 40 is provided with the mating connector member 41 that is configured to be connected to the connector port member 20 of the dispenser tube 15. A closure can be present on the outlet of the static mixer and/or on the outlet of each of the chambers 31, 32 of the double barrel syringe injector 30.

The figure 1 illustrates that the injector 30 which is filled with the liquid antimicrobial coating substance and with the solvent and/or diluent is also accommodated in the outer packaging 1, here in storage recess 5 of the tray 2. In another embodiment, the injector 30 which is filled with the liquid antimicrobial coating substance and possibly also with the solvent and/or diluent is packaged separate from the outer packaging 1 containing the catheter 10 which is received in the dispenser tube 15.

It is envisaged that the hermetically sealed outer packaging 1 containing the catheter 10 received in the dispenser tube 15 as well as the injector 30 is subjected to a sterilization process, e.g. comprising an irradiation step, so that the entire packaged system is sterilized.

In use, shortly before the actual introduction of the catheter 10 into a patient, the outer package 1 can be opened.

The injector 30 is connectable to the connector port member 20 allowing the dispensing of the liquid antimicrobial coating substance from the injector 30 so as to introduce the liquid antimicrobial coating substance into the dispenser tube 15 thereby coating the exterior of the catheter 10 with the liquid antimicrobial coating substance prior to removal of the coated catheter 10 from the dispenser tube 15. In the depicted version, the antimicrobial coating substance is mixed with solvent and/or diluent in the static mixer 40, with the mixture then being dispensed into the dispenser tube 15.

For example, between 10 ml and 30 ml of liquid antimicrobial coating substance is held in the chamber 31.

The substance, possibly mixed with solvent and/or diluent, is preferably easily flowable, so that the substance flows from the rear end 17 towards the front end 16 of the dispenser tube 15. Possibly, the dispenser tube 15 is appropriately manipulated to enhance the distribution of the substance over the length thereof.

As the liquid antimicrobial coating substance enters into and spread over the length of the dispenser tube 15 in which the catheter 10 is still present, the exterior of the catheter 10 becomes coated with the substance.

Once externally coating of the catheter 10 has been effected, the catheter 10 is removed from the dispenser tube via the front end thereof. As explained, it is preferred to effect this process of externally coating the catheter 10 briefly before the actual insertion of the catheter 10 into the patient.

As discussed herein, in a preferred embodiment, the liquid antimicrobial coating substance comprises propolis. The propolis containing external coating of the catheter 10 may provide enhanced protection against catheter related infections.

## Claims

1. A catheter system comprising:
- an outer packaging (1) containing therein a catheter (10) which is received in a dispenser tube (15), the catheter having an exterior,
- a manually operable injector (30) which is filled with a liquid antimicrobial coating substance,
wherein the dispenser tube (15) is provided with a connector port member (20), and
wherein the injector is provided with a mating connector member (41), that is connectable or connected to the connector port member (20) allowing the dispensing of the liquid antimicrobial coating substance from the injector (30) so as to introduce the liquid antimicrobial coating substance into the dispenser tube (15) thereby coating the exterior of the catheter (10) with the liquid antimicrobial coating substance prior to removal of the coated catheter (10) from the dispenser tube (15).

2. The catheter system according to claim 1, wherein the liquid antimicrobial coating substance comprises propolis.

3. The catheter system according to claim 1 or 2, wherein the connector port member (20) is provided at an end (17) of the dispenser tube.

4. The catheter system according to any one or more of claims 1 - 3, wherein the connector port member (20) comprises a Luer fitting, e.g. a female Luer fitting, e.g. a female Luer - lock fitting, and wherein the injector comprises a mating Luer fitting, e.g. a male is a Luer fitting, e.g. a male Luer - lock fitting.

5. The catheter system according to any one or more of claims 1 - 4, wherein the injector (30) comprises a first chamber (31) which is filled with the liquid antimicrobial coating substance and a second chamber (32) which is filled with a solvent and/or diluent, e.g. alcohol, and wherein the injector (30) is configured to dispense from both the first and second chambers (31,32) simultaneously.

6. The catheter system according to claim 5, wherein the injector comprises a static mixer (40) which is connected or connectable to the first and second chambers (31,32) at an inlet of the static mixer (40), wherein an outlet of the static mixer is provided with the mating connector member (41).

7. The catheter system according to any one or more of claims 1 - 6, wherein the injector (30) is a syringe, e.g. a double barrel syringe having a first chamber (31) which is filled with the liquid antimicrobial coating substance and a second chamber (32) which is filled with a solvent and/or diluent.

8. The catheter system according to any one or more of claims 1 - 7, wherein the injector (30) holds between 10 ml and 30ml of the liquid antimicrobial coating substance.

9. The catheter system according to any one or more of claims 1 - 8, wherein the outer packaging (1) is embodied as a flexible pouch or as a tray (2) covered by a foil.

10. The catheter system according to any one or more of claims 1 - 9, wherein the manually operable injector (30) filled with the liquid antimicrobial coating substance is initially contained within the outer packaging (1).

11. A method for preparing an externally coated catheter, wherein a system according to one or more of the claims 1 - 10 is provided, and wherein the method comprises:
- opening the outer packaging (1),
- possibly, connecting the injector (30) with the mating connector member (41) thereof to the connector port member (20) of the dispenser tube (15),
- operating the injector (30) so as to dispense the liquid antimicrobial coating substance from the injector so as to introduce the liquid antimicrobial coating substance into the dispenser tube (15) thereby coating the exterior of the catheter (10) with the liquid antimicrobial coating substance prior to removal of the coated catheter from the dispenser tube,
- removing the coated catheter from the dispenser tube (15).
